# EUROPEAN PATENT APPLICATION

(11) **EP 2 184 039 A1**
(43) Date of publication of application: **12.05.2010**
(21) Application number: 08398013.6
(22) Date of filing: 11.11.2008
(51) Int. Cl.: A61F 9/06

(54) **Welding helmet with optimized ventilation system**

(71) Applicant: EWF - European Federation for Welding, Joining and Cutting Secretariat, 2780-994 Porto Salvo (PT)
(72) Inventor: Nilberto, Alessandro, 17011 Albisola Superiore (SV) (IT)

(57) **Abstract**

The present invention, titled **Welding Helmet with Optimized Ventilation System** relates to a new type of welding helmet that features an innovative ventilation system that greatly improves the conditions in which a welder performs his work.

The principal aim of this invention is to provide a solution to one of the great hazards of the welding activity, **i.e.,** the inhalation of hazardous welding fumes by the welder, leading to severe health complications.

This ventilation system is characterized by providing a substantial increase in the air circulation to the welder's face that not only does it not hinder the welder's activity but also prevents the actual inhalation of the welding fumes by the welder. It does this through an innovative design of the welding helmet, consisting of the addition of two improved lateral diffusers, placed in a lowered and rear position in order to prevent the diffusion of the welding fumes, to the main body of the helmet, through which the air is made to circulate, introducing a fresh air current at the level of the welder's chin.

The use of this particular welding ventilation system would allow for greatly improved health conditions for those involved in welding activities in confined spaces, or where are not available suitable systems for the fume extraction, where is greater the risk of inhalation of welding fumes.

## Description

### 1. Field of the Invention

This invention relates directly to the welding activity, specifically with regards to the use of ventilated welding helmets during the welding process. The invention, in particular, pretends to deal with an innovative ventilation system to be applied to the welding helmet, thus enabling the welder to work in a completely fume-free environment.

Such an achievement is of considerable importance when working in confined spaces or where are not available other suitable systems for the fume extraction.

### 2. Description of the Prior Art

The issue of welding fumes has been a constant problem of the welding activity, one that greatly contributes to the list of problems that affect welders in general, during their work. Evidently, fume emission is of particular concern for the welder, due to the high levels of toxicity that these fumes possess and which will, eventually, contaminate the welder.

The consequences deriving from the welder's exposure to different concentrations of welding fumes have been substantially demonstrated and documented, and the seriousness of this matter cannot be stressed enough. Indeed, fume emissions during welding vary depending on the materials involved, with results varying between short and long term influence, like Parkinson-like diseases as well as potential carcinogenic diseases.

Efforts to counter these and other problems of the welding activity have been substantial and varied in their different approaches, particularly in terms of the welder's personal protection, of which the welding helmet plays a central role. Consequently, previous models of ventilated welding helmets have tried to counter not only the fume emission coming from the welding activity, but also the intense flash resulting from the use of the different welding techniques, and its effect on the welder's eyes.

However, it can also be said that only recently can the specific issues of welding fumes emissions, namely efforts towards its prevention, taken a significant hold and, as a consequence, the prior art in this field does not appear extensive. One such example is US4293757 issued October 10th 1981, which allows for a dark eye shield to be automatically moved into position, relative to the welder's mask, while also describing the possibility of a ventilation system resulting from the pneumatic air that operates the dark eye shield, resulting in the washing of welding fumes and overall cooling of the welder's head. Nevertheless, this particular invention as well as previous ones that can be mentioned, like US3096430 and US2761046 primarily focus on the issue of allowing some sort of shielding for the welder's eyes, not taking into consideration fume emission issues, except in the case of the already mentioned US4293757 that, however, presents severely outdated concepts in order to implement the air circulation and subsequent fumes reduction.

The present invention presents an innovative alternative to the problem of fume emission by employing a new ventilation system applied to the welder's helmet.

As a result of the performance of the European project Econweld (COLL-CT-2005-516336) it was found out that the 'normal' ventilated helmets do not ensure against the introduction of welding smoke into the mask from its bottom.

This finding is clearly described in the Deliverable Report D 3.3 'Design of smart helmet with optimized air flow system'.

Such a report states the goal to design a new kind of ventilated helmet with a modified design of the air supplying ducts in such a way that the introduction of welding smoke into the helmet from its bottom is prevented.

This objective has been reached by implementing two extended lateral diffusers, suitably placed in a lowered and rear position in the helmet shell, through which a given quantity of fresh air is made to circulate and diffused at the level of the welder's chin.

In particular, the basic concept implemented in the new ventilated helmet design, here proposed, achieves the goal of insulating the helmet inner volume from the welding fumes diffusion by means of an air blade which while allowing the expulsion of the inner air toward the outside also replaces it by fresh air supplied by an additional air inlet placed in the top side of the ventilated helmet (refer to Deliverable Report D3.3 "Design of a Smart Helmet with Optimized Air Flow System", ECONWELD Project).

The implementation of this new ventilation system results in a really new concept of welding helmet, presenting a significantly modified design of the fresh air supplying system and air-ducts. Such a new kind of welding helmet, in virtue of its functioning and constructional principle, really guarantees against the introduction of welding smoke into the mask from its bottom.

In such a way this new device realizes a great improvement upon the existing technology and know-how.

### 3. Brief Description of the Drawings

Taking into consideration the problems the present invention means to solve, that are well described in the above cited deliverable report Del D3.3 "Design of smart helmet with optimized air flow system" of the Econweld Project, these will be better understood through referencing to the following drawings, showing the main components of the welding helmet:
FIG.1 shows the helmet's main air duct.
FIG.2 shows the modified lateral air diffusers.
FIG. 3 shows the helmet's face lodging.
FIG.4 shows a general view of the helmet and the welder's face.
FIG. 5 provides an internal view of the helmet shell.
FIG.6 provides a general view of the new concept lateral air ducts.

The principle of the functioning of new device is clearly described into the Deliverable Report D 3.3 'Design of a smart helmet with optimized air flow system of the above said Econweld Project.

The present invention, in effect enables fume prevention free of added discomfort to the welder while performing his job. This achievement is due to the fact that the air flow recirculation within the helmet doesn't impinge on the welder face with a sensible velocity nor it has a significant sliding, in bottom up direction, on the welder face.

Those findings are the result of the performance of a deliverable (Del D3.3 "Design of smart helmet with optimized air flow system") of the European Research Project Econweld (COLL.CT-2005-516336). In such deliverable an accurate fluid-dynamics modelization of the air behavior at the inside of the helmet shell has been executed.

### 4. Description of the preferred embodiment

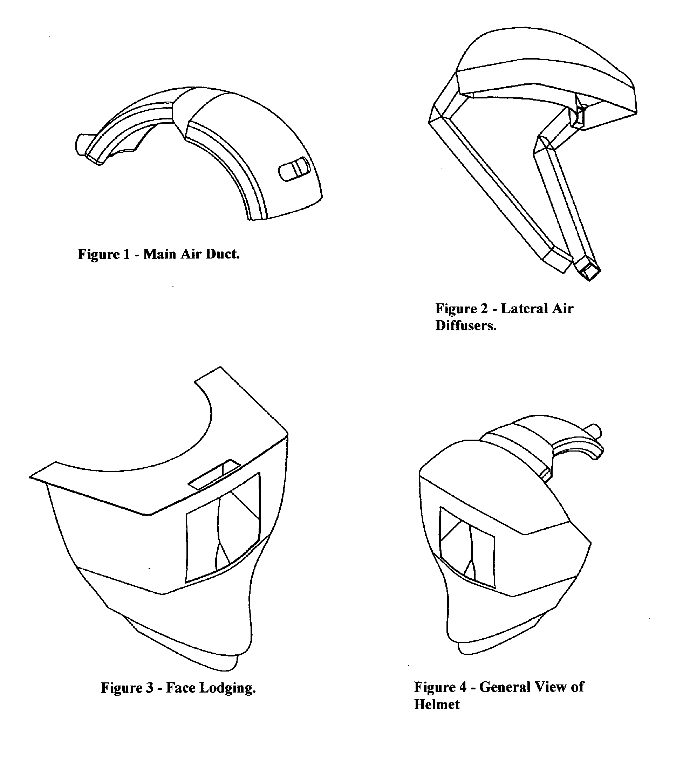

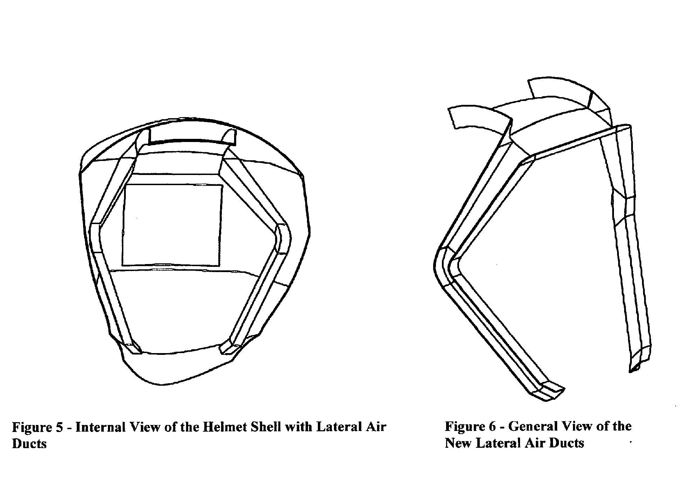

Having as reference the above mentioned figures, the welding helmet is composed of the more common face lodging (Fig.3) with the appropriate visor, as well as the helmet's main air duct (Fig.1) providing for the circulation of air through said helmet. However, as shown in Fig.2 and Fig. 6, the addition of the lateral diffusers represents an improvement that goes beyond the present conventional welding helmets. These lateral diffusers are positioned based on an improved geometry concept, lowered and placed at the rear of the welding helmet The resulting welding helmet, as shown in Fig.4, will allow for a complete prevention of welding fumes inhalation, while also ensuring an effective fresh air ventilation to the welder's face, without causing discomfort to the welder, as the air flow doesn't impinge on the welder face with a sensible velocity nor it has a significant sliding, in bottom up direction, on the welder face.

### 5. Exploitation in Industry

We think that the new invention can be put in production in such a way to provide a better level of health and safety conditions in working situations where a welder is obliged to operate in confined spaces, or where other suitable systems for the fume extraction are not available.

## Claims

1. A new welding helmet **characterized by** an improved air ventilation system.

2. A welding helmet as set forth in claim 1, wherein said ventilation system is **characterized by** the inclusion of two lateral diffusers to the main body of the welding helmet.

3. The welding helmet as set forth in claim 2, wherein the said lateral diffusers are arranged according to an innovative geometric concept, **characterized by** the emission of fresh air at the base of the welder's chin, thus allowing for the prevention of the inhalation of welding fumes. Such achievement is the result of the performance of a deliverable (Del D3.3 "Design of smart helmet with optimized air flow system" of the European Research Project Econweld (COLL.CT-2005-516336). In such deliverable accurate fluid-dynamics modelizations of the air behavior at the inside of the helmet shell has been executed, allowing for validation of the functionality of the innovative ventilated helmet.
